# EUROPEAN PATENT APPLICATION

(11) **EP 2 109 055 A1**
(43) Date of publication of application: **14.10.2009**
(21) Application number: 08154441.3
(22) Date of filing: 11.04.2008
(51) Int. Cl.: G06F 19/00

(54) **Portable psychological monitoring device**

(71) Applicant: Universiteit Maastricht, 6211 LK Maastricht (NL); Academisch Ziekenhuis Maastricht, 6229 HX Maastricht (NL)
(72) Inventor: Delespaul, Philippe Armand Etienne Ghislain, B-3630 Maasmechelen (BE); van Os, Johannes Jacobus, 6224 GC Maastricht (NL); de Vries, Marten William, 6211 KB Maastricht (NL); Nicolson, Nancy Anne, 6211 KB Maastricht (NL); Edmonds, Peter Maria Joseph, 6225 CM Maastricht (NL); Germeys, Inez, B-3740 Bilzen (BE); Peeters, Franciscus Petrus Maria Ludovicus, 6211 HM Maastricht (NL); Wichers, Maria Catharina, 6224 GC Maastricht (NL)
(74) Representative: Hatzmann, Martin

(57) **Abstract**

The invention relates to a portable psychological monitoring device, comprising an input, an output, a repository and a processor. The repository comprises a plurality of questionnaires, each comprising one or more questions relating to a mental state of a user. The processor is arranged for selecting a first questionnaire of the plurality of questionnaires, and presenting at least one of the questions of the first questionnaire to the user via the output at a first moment that is random to the user. The processor is arranged for receiving an answer to the question from the user via the input, and storing the answer into the repository. The invention further relates to a system for psychological monitoring, a method of assessment of a mental state of a user and a method of treatment or prevention of mood disorders and related disorder phenotypes.

## Description

### Field of the invention

The invention relates to psychotherapy, psychological monitoring, more specifically to the contextual monitoring of mental state and behaviour. Applicability is to assess and to induce changes in human mental state and behaviour through feedback.

Fields of application are general and specialised medicine and psychology, as well as the area of well-being.

### Background to the invention

### Experience Sampling Method

The development of Behavioural Monitoring has been frustrated by challenges to reliability (or the assessment of reliability) when subjects have to collect private information (mental state reports) that by definition cannot be assessed independently. Equally challenging is the report of individual data that could be checked through external observation (e.g. what people do, where they are, who they're with), but occurs in places were no external observatory is available.

Over the years researchers have developed assessment technology that allows the reliable and valid collection of behavioural content in an ecological context, throughout the normal daily living environment of the individual. They standardized the protocols and have generated consensus about the conditions that reduce assessment reactivity (challenges to validity and reliability) and the relevant functionality of the supporting instrument. These methods have been published using different names: Experience Sampling Method (ESM), Ecological Momentary Assessment (EMA), Beeper studies or behavioural monitoring (non-exhaustive list), herein below generally referred to as Experience Sampling (Method) or ESM.

Experience Sampling Methods have primarily been applied in university settings and experimental clinical conditions. Transportability of the assessment technology without concessions to the reliability and validity of the instrument has been difficult. Experience Sampling relies on complex instructions and often generates a large data flow that is difficult to manage.

Over the years the Maastricht centre standardized the protocols and reached consensus about the relevant functionality. A hardware and software solution that reduces the threshold to apply this assessment methodology in normal clinical practice is highly needed.

### Ecological Behavioural Monitoring and Feedback (E-BMF)

Behavioural Monitoring and Feedback (BMF) is an essential instrument for inducing changes in human behaviour. For example, BMF is an essential element in many evidence-based psychotherapies and key to Behaviour Modification programmes. However, BMF typically is difficult and expensive to apply in clinical practice, as it relies on cross-sectional instructions of the person, e.g. patient, who is then left to execute a complicated series of monitoring exercises without supervision or reminders. In addition, BMF in this context relies on retrospective judgements on coarse descriptions of emotion or behaviour with minimum room for the assessment of context and order of experiences. Therefore, BMF, although successful for some conditions requiring systematic exposure and monitoring of simple reactions (e.g. phobic disorders), remains insufficiently developed to assess, monitor and change the full range of human experiences in the flow of daily life. In addition, BMF is only successful in the hands of highly specialised, and therefore expensive, therapists that can only reach a minority of those who need it.

Most, if not all somatic disorders are influenced by mental processes associated with regulation of emotions and behaviour through well-known interactions with the immune, endocrine, coagulatory, gastro-intestinal, cardiac and central nervous systems. Although the field of behavioural medicine has developed interventions to, for example, reduce anxiety and avoidance in cardiac patients, increase acceptance in end-of-life situations or reduce stress in patients with stress-related conditions, the fact remains that somatic medicine is almost entirely devoted to treating somatic conditions with little or no attention to the emotional and behavioural processes that codetermine the onset and the outcome of the disorder in question. The reason for this is related to the treatment capacity of mental health services: it is simply impossible to practise true integrated medicine, as the costs would be too high. An example is conditions such as anxiety and depression, the great majority of which are treated with psychiatric medications, whereas it is well known that the combination of antidepressant medication and psychotherapy is much more effective than the sum of their effects alone.

There is an urgent need for the widespread and generalised use of BMF techniques in the areas of medicine, psychology and well being that go beyond the simple cross-sectional approach of assessing human emotions and experience.

Crucial for the success of these BMF interventions is the applicability outside of the standardized medical context (e.g. the office of the psychotherapist) into the normal daily living context of patients. This is the place where patients actually experience and suffer from their mental problems. It is the place, where the effect of BMF should be transported to reach stable and generalized behavioural change. Therefore BMF in this context is an Ecological BMF (E-BMF).

### Brief Description of the Invention

A goal of the invention is to allow simple and effective monitoring of the mental state of a person in the flow of daily life.

Thereto, according to the invention, a portable psychological monitoring device is provided, comprising an input, an output, a repository and a processor, wherein the repository comprises a plurality of questionnaires, each comprising one or more questions relating to a mental state of a user, wherein the processor is arranged for selecting a first questionnaire of the plurality of questionnaires, and presenting at least one of the questions of the first questionnaire to the user via the output at a first moment that is random to the user, and wherein the processor is arranged for receiving an answer to the question from the user via the input, and storing the answer into the repository.

Said device allows for the assessment of moment-to-moment human experiences in the flow of daily life, allowing not only for the assessment of complicated experiences including affective state, stress, salience, motivation, pain and many others, but also of the context and/or order in which the experience arose, allowing for the assessment of human responses in the flow of daily life. This is mainly due to the interruption of the flow of daily life by the device at the first moment in time which is random to the user, i.e. which is unknown to the user, so that the device surprises that user in his or her daily flow of life with presenting the question. Hence, the answer to the question will be much less retrospective and will provide much more valuable information on the momentary psychological state of the user.

Herein questionnaire may be interpreted broadly, a questionnaire may comprise one or more questions, one or more tasks or a combination thereof.

Preferably, the processor is arranged for selecting a second questionnaire of the plurality of questionnaires and presenting at least one of the questions of the second questionnaire to the user via the output at a second moment that is known to the user. Thus, it is possible to provide an event-triggered questionnaire to the user at a moment that is known to the user, in addition to the questionnaire presented to the user at the moment that is random to the user. Because event sampling can generate response attrition the event sampling procedure may be provided with a randomization option, so that mutually different questionnaires may be presented to the user at similar (or identical) events.

Preferably, the first moment in time and/or the second moment in time (and/or further moments in time) are stored in the repository. Preferably, the repository comprises information regarding a time schedule according to which one or more questionnaires are to be presented to the user. Preferably, the device is arranged for adjusting a density of sampling over time, i.e. the number of questionnaires presented to the user per unit time, for example 10 times per day for 7 consecutive days, to 2 times per day for 14 consecutive days, to 5 times per day for 35 consecutive days. Depending on the density of momentary sampling used, the actual course of the experience over time can be depicted in great detail.

There is virtually no limit in the type of human experience/response that can be sampled.

In an embodiment, the device is arranged for changing the momentary assessment protocol during the momentary sampling period, e.g. through remote programming of the device by an operator such as a therapist, in response to changes in user's state. Here the momentary assessment protocol may e.g. be changed so as to select a different questionnaire or to present to the user a (different) exercise. Also, the assessment protocol may be changed by changing the density of sampling. Here an assessment protocol is to be understood as the protocol defining the density of sampling and the questionnaires presented to the user.

In an embodiment, the device is arranged to monitor physical, physiological and/or biochemical parameters of the user. Thereto, the device may be provided with sensors to measure a value of such parameters, such as blood pressure, heart rate, body temperature, etc. Alternatively, or additionally, the device may be provided with connections (e.g. sockets and/or wireless connections) to communicatively connect the device to external sensors. Preferably, the parameters are monitored during answering a questionnaire. The parameters may also be monitored during a period of time before the questionnaire is presented to the user, and/or after the questionnaire has been completed by the user. Hence, information obtained from the questionnaire may be combined with information obtained from monitoring the parameters at substantially the same moment in time. This combined information increases the options for an operator, e.g. a physician, to assess the mental state of the user.

Optionally, the device is designed to have appeal, such as a life-style article, which may further enhance user compliance with the assessment protocol. Optionally, the device is provided with additional functionality such as time display.

Preferably, the device is arranged as an interactive device. This means that rather than awaiting whether or not the user complies with the assessment protocol, the device is arranged to actually request, e.g. using a human voice signal, that the user provides the device with the necessary input. This will greatly increase compliance with the assessment protocol.

Preferably the device is arranged such that it can always be carried with the user and therefore can never be mislaid. The device may e.g. be provided with a clip for attaching the device to a belt or clothing. The device may also be of "pocket-size", i.e. be sufficiently small to be carried in a pocket of e.g. a pair of trousers. The device may also be provided with a strap, e.g. a wrist-strap, to allow the device to be worn on a body part, e.g. a wrist, like a wristwatch.

For example, the device can be arranged to be worn as a wristwatch with extremely simple input controls adapted for use by children, adults and the elderly.

In an embodiment, the device is arranged to provide feedback, on the basis of data entered by the user in response to one or more questions of a questionnaire, a questionnaire or a succession of questionnaires. Preferably, the device is arranged to present the feedback to the user, e.g. via the output of the device. Alternatively, or additionally, the device may be arranged to transmit the feedback to the operator, e.g. the therapist, e.g. through a wireless communications transmission. The device may also be arranged to transmit data inputted into the device by the user to the operator, e.g. via a wireless or wired communications link.

In an embodiment, the device is arranged to, preferably automatically, generate output reports indicating behavioural changes. Such output records may e.g. be transmitted to a processing station, such as a general purpose computer.

The device may be provided with a transmitter for the above mentioned transmissions. The transmitter may be arranged to communicate via USB, Firewire, Ethernet, Bluetooth, ZigBee, GSM, GPRS, UMTS, HSDPA, EDGE, WLAN, WiMax, or other suitable wired or wireless communications protocol.

The invention further relates to a system for psychological monitoring, comprising a portable psychological monitoring device according to the invention, an operator station, and optionally a user station, wherein the portable device is arranged for transmitting data relating to the mental state of the user to the operator station, optionally via the user station.

The invention further relates to a method of assessment of a mental state of a user, comprising providing a device according to the invention to the user, selecting a questionnaires comprising one or more questions relating to a mental state of a user, having the device present at least one of the questions of the questionnaire to the user at the first moment that is random to the user, and receiving an answer to the question from the user using the device. It has been found that said method allows for the assessment of moment-to-moment human experiences in the flow of daily life, allowing not only for the assessment of complicated experiences including affective state, stress, salience, motivation, pain and many others, but also of the context and/or order in which the experience arose, allowing for the assessment of human responses in the flow of daily life.

More specifically, the invention relates to a method of assessment of a mental state of a person having mood disorders and related disorder phenotypes and being subjected to psychotherapy, possibly Cognitive Behavour Therapy or Mindfulness Based Therapy, using a portable psychological monitoring device according to the invention. It has been found that using said method for these persons provides the effect that the use of the device greatly enhances effectiveness of the applied psychotherapy.

Further, the invention relates to a method of treatment or prevention of mood disorders and related disorder phenotypes, in a person in need thereof, comprising subjecting the person to psychotherapy, possibly Cognitive Behaviour Therapy or Mindfulness Based Therapy, using a portable psychological monitoring device according to the invention, directed to improving the person's ability to experience reward

### Brief Description of the Drawing

The invention is further elucidated by means of non-limiting examples. Here is referred to the drawing in which
Fig. 1 shows an example of a first embodiment of a portable psychological monitoring device according to the invention;
Fig. 2 shows an example of a second embodiment of a portable psychological monitoring device according to the invention;
Fig. 3 shows an example of a third embodiment of a portable psychological monitoring device according to the invention;
Fig. 4 shows an example of a flow chart of a process according to the invention;
Fig. 5 shows an example of a protocol file;
Fig. 6 shows an example of a questions file; and

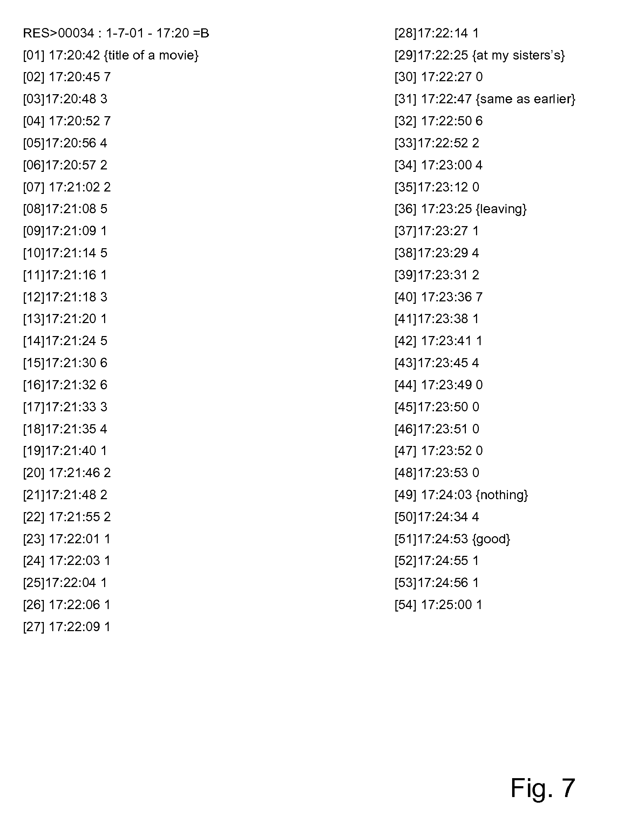

Fig. 7 shows an example of a results file.

### Detailed Description of the Invention

### Mood Disorders and Related Phenotypes

Although a wider applicability of the present invention is not excluded, the invention may in particular be practiced in treating or preventing mood disorders and related phenotypes. This includes depression, including sub-threshold depression, dysthymia, mood disorder due to a general medical condition, substance-induced mood disorder, adjustment disorder, phobic disorder and addiction (substance abuse or dependence).

### Reward Experience

This relates to the so-called positive affect, which is a known concept in psychology. It refers to a person's ability to experience positive emotions from everyday life situations as the effect of minor events on positive affect in the flow of daily life. This reward experience can be quantified e.g. using the Experience Sampling Method (ESM). ESM is a structured diary technique to assess subjects in their daily living environment, and has been extensively validated for the use of immediate effects of stressors on mood

With reference to the definition and quantification of reward experience, it is noted that the invention is not limited to just mental processes. Rather, the invention provides a concrete and novel tool to treat or prevent mental state disorders and related phenotypes, by bringing about an improvement in a person's reward experience.

The concept of reward experience is literally linked to a person's experience, and therewith not to be derived from any existing brain function models. It should not be mistaken for a purely mental process, as the result of steering a person's ability to experience reward, if successful in bringing about an improvement thereof, may also be a pharmacological process. This process can be mediated through psychotherapy, through pharmacotherapy, and preferably through both, most preferably simultaneously.

### Reward-related mental disorders

In addition to the use of a person's ability to experience reward as a tool to therapy or prevention as described above, the invention also relates to the treatment or prevention of mental disorders that can be defined as being related to reward. These can be described as disorders associated with alterations in the ability to experience reward, hereinafter "reward disorders," abbreviated as RD.

RD denotes a group of disorders which can be readily distinguished from mental disorders as regularly defined (such as, e.g. Major Depressive Disorder (MDD) and Substance Abuse) by the additional clinical component of inability, or diminished ability, to experience reward. Thus, with any given patient diagnosed with any one disorder within said regular groups of disorders, the recognition of a diminished ability, or even inability, to experience reward, is capable of working as a tool to define proper therapy.

E.g., a patient diagnosed with MDD may or may not have benefit from anti-depressant therapy of whichever kind. However, a patient diagnosed with MDD for whom it can be assessed that he or she should in fact be diagnosed with RD, will likely benefit more from therapy treating the RD, rather than from traditional anti-depressant therapy.

### Portable psychological monitoring device

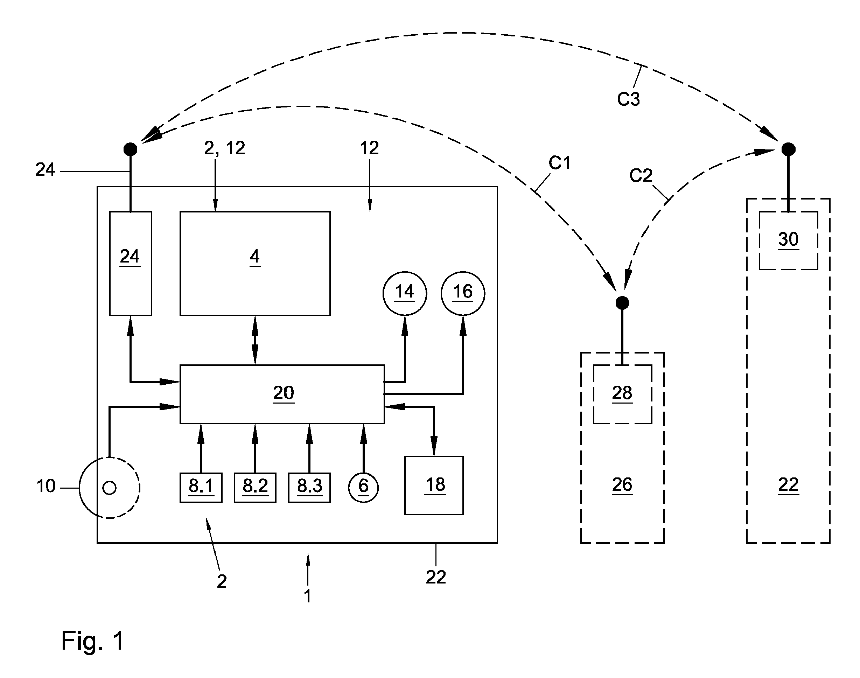

According to the invention, a portable psychological monitoring device is provided. Fig. 1 shows a schematic representation of an example of a first embodiment of the portable psychological monitoring device 1.

The portable psychological monitoring device 1 comprises an input 2, in this example formed by a touch screen 4, a microphone 6, a plurality of control buttons 8.i (i=1,2,3...) and a scroll wheel 10. The device 1 further comprises an output 12, in this example formed by the touch screen 4, a speaker 14 and a vibratory element 16. The device 1 further comprises a repository 18, here formed by a non-volatile memory, and a processor 20. In this example, all components are housed in a housing 22.

In this example the repository 18 comprises a plurality of questionnaires, each comprising a plurality of questions relating to a mental state of a user, e.g. a patient. The processor 20 is arranged for selecting a first questionnaire of the plurality of questionnaires, and presenting at least one of the questions of the first questionnaire to the user via the output 12, e.g. in writing on the touch screen 4 and/or spoken via the speaker 14. In this example, the processor is arranged to present the selected questionnaire in full, i.e. all questions in the selected questionnaire will be presented to the user to be answered. It will be appreciated that it is possible that the processor 18 is arranged to refrain from presenting certain questions in the selected questionnaire to the user, e.g. if an answer to a previous question obviates the need or use of presenting such subsequent question to the user.

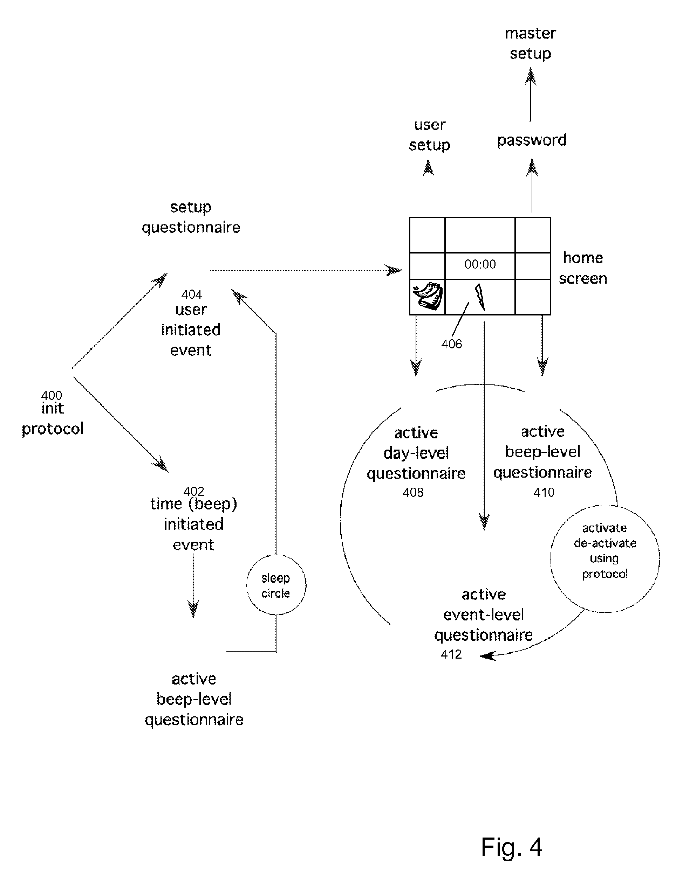

In this example the memory 18 comprises data representative of a first moment in time at which the question of the first questionnaire needs to be presented to the user. The processor 20 is arranged to present the question to the user at said first moment (see step 402 in Fig. 4). Here the first moment is predetermined and may e.g. be stored into the memory by an operator, such as a physician or technician. It will be appreciated that the first moment is random to the user, in that the user is unaware of time represented by the first moment, so that the question will be presented to the user when he is not expecting the question to be presented, i.e. the user is surprised by the device 1 presenting the question. Thus, the answer to the question will be much less retrospective and will provide much more valuable information on the momentary psychological state of the user than when the user knew in advance that the question would be presented at that very moment.

Here, the processor 20 is further arranged for receiving an answer to the question from the user via the input 2, and storing the answer into the memory 18. The user may e.g. input the answer by speaking into the microphone 6, "clicking" a selected answer on the touch screen 4, by pressing one or more buttons 8.1 and/or by making a selection via the scroll wheel 10. It is also possible that the user types the answer (e.g. in the event of an open question), e.g. using a (e.g. alphanumerical) keyboard presented on the touch screen 4.

Alternatively, or additionally, the processor is arranged to select a second questionnaire of the plurality of questionnaires, and presenting at least one of the questions of the second questionnaire to the user via the output 12, while the memory 18 comprises data representative of a second moment in time at which the question of the second questionnaire needs to be presented to the user. The processor 20 is arranged to present the question to the user at said second moment. Here the second moment is predetermined and known to the user, and may e.g. be stored into the memory by a physician or technician. Therefore, the second questionnaire may be presented to the user at a fixed moment in time, which is known to the user. The second questionnaire may e.g. be presented to the user each morning and/or each evening. The second questionnaire may also be presented to the user upon request by the user (see step 404 in Fig. 4). Also in this case the second moment at which the second questionnaire is presented to the user is known to the user.

It will be appreciated that the first questionnaire and the second questionnaire are presented to the user at different types of moments. The first questionnaires are presented to the user at the first moment that is random to the user, whereas the second questionnaire is presented the second moment that is known to the user. It will be appreciated that the content of the first and second questionnaire (e.g. the questions) need not necessarily be different.

In this example, the device 1 is arranged as an interactive device. I.e. the device 1 is arranged to actually request, e.g. using a human voice signal, that the user provides the device with the necessary input, e.g. the answer to a question. This greatly increases compliance with the assessment protocol.

The device 1 shown in Fig. 1 is arranged to provide feedback, on the basis of data entered by the user via the input 2 in response to one or more questions of a questionnaire, a questionnaire or a succession of questionnaires. In this example, the device 1 is arranged to present the feedback to the user, in the form of readable information, such as words, a graph and/or a picture, via the touch screen 4 of the device 1. In this example, the device 1 is further arranged to transmit the feedback to an operator station 22, e.g. a general purpose computer, of the operator. In this example the device 1 is also arranged to transmit data inputted into the device 1 by the user to the operator station 22. In this example the device 1 comprises a transmitter 24 for transmitting the feedback and/or data. In this example, the transmitter 24 is arranged to communicate with a user station 26, e.g. a general purpose computer, of the user via a wireless communications connection (indicated by C1 in Fig. 1), such as Bluetooth. Thereto the device 1 further comprises an antenna 26 associated with the transmitter 24. The user station 26 comprises a receiver 28 for receiving the feedback and/or data from the device 1. In this example, the user station 26 is arranged to relay the feedback and/or data to the operator station 22, e.g. via email or internet. Thereto, in this example, the receiver 28 is designed as a transceiver. Alternatively, the user station 26 may be provided with a separate transmitter. Further, the operator station 22 is provided with a receiver 30 for receiving the feedback and/or data from the user station 26 via the wired or wireless communications connection C2.

It will be appreciated that alternatively, or additionally, the device 1 may be arranged to directly transmit the feedback and/or data to the operator station 22 via a direct, optionally wireless, communications connection C3 such as Bluetooth, GSM, GPRS or the like.

In this example the feedback may comprise information, such as instructions, reassurance, comforting words and/or additional tasks or questionnaires. The feedback may be provided to the user with the intention to alter a current mental state into a desired mental state.

In this example, the device 1 is arranged for adjusting a density of sampling over time, i.e. the number of questionnaires presented to the user per unit time. Thereto, an indication of the moment at which a questionnaire is to be presented to the user, and optionally an indication of which questionnaire is to be presented at that moment may be stored into the memory 18 of the device 1. By storing more indications of moments at which a questionnaire is to be presented, the density of sampling may be increased.

In this example, the device 1 is arranged for changing the momentary assessment protocol during the momentary sampling period. Here the processor 20 is arranged to change the momentary assessment protocol so as to select a different questionnaire or to present to the user a (different) exercise, e.g. in response to an answer to one or more previous questions, the results of a previous exercise and/or the outcome of one or more previous questionnaires. The device 1 may be arranged to automatically change the momentary assessment protocol, e.g. based on a predefined set of rules, stored in the memory 18. Alternatively, or additionally, the momentary assessment protocol may be changed through remote programming of the device 1 by an operator such as a therapist, e.g. in response to an answer to one or more previous questions, the results of a previous exercise and/or the outcome of one or more previous questionnaires, the results of which have been transmitted to the operator station 22 via the communications connections C1 and C2 or C3 as elucidated hereinabove. The receiver 30 of the operator station 22 may be arranged as a transceiver, or the operator station 22 may comprise a separate transmitter, for transmitting a control signal to the device 1 for changing the assessment protocol. The device 1 then comprises a receiver, or the transmitter 24 is arranged as a transceiver, for receiving the control signal via the communications connections C2 and C1 or C3.

In the example of Fig. 1, the device 1 is arranged to, preferably automatically, generate output reports indicating behavioural changes. Such output records may e.g. be transmitted to a processing station, such as the user station 26 or the operator station 22. In this example, the transmitter 24 is arranged to also transmit the output reports to the user station 26 and/or the operator station 22. It will be appreciated that it is also possible that the output report indicating behavioural changes may be generated by the user station 26 and/or the operator station 22, thereto provided with a suitable software code portion, on the basis of data received from the device 1.

It will be appreciated that the device 1 in combination with the operator station 22, and optionally the user station 26, forms a system for psychological monitoring.

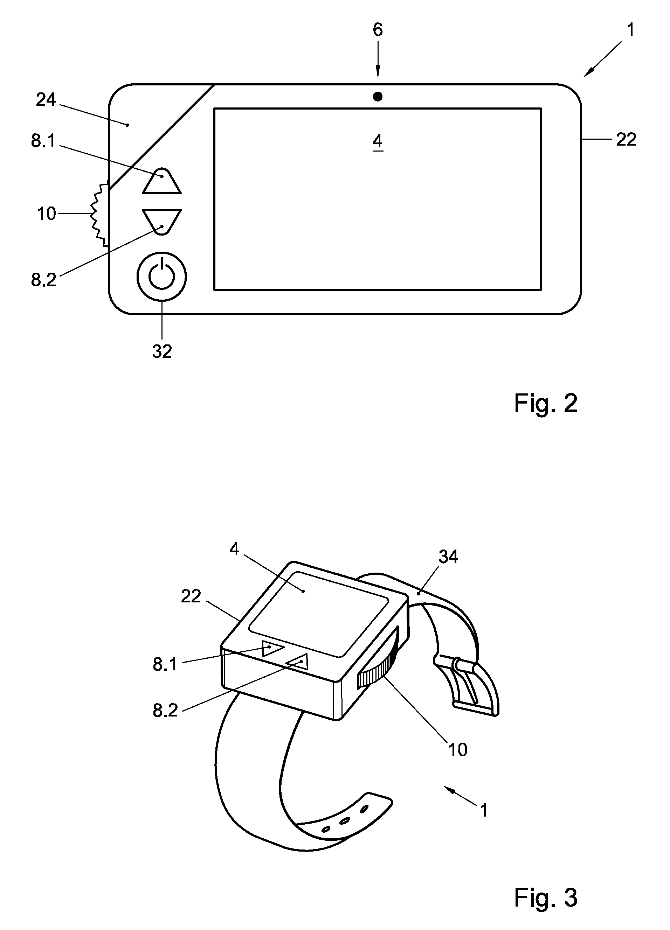

Figs. 2 and 3 show examples of further embodiments of a portable psychological monitoring device 1 according to the invention. The device 1 shown in Figs. 2 and 3 comprises the same functionality as the device 1 shown in Fig. 1 unless otherwise noted. Parts internal to the housing 22 may not be visible in Figs. 2 and 3.

In Figs. 2 and 3 the device 1 is designed to have appeal, such as a life-style article, which may further enhance user compliance with the assessment protocol. In the examples of Figs. 2 and 3, the device 1 is arranged such that it can always be carried with the user and therefore can never be mislaid.

In the example of Fig. 2, the device 1 is designed to be of "pocket-size", i.e. sufficiently small to be carried in a pocket of e.g. a pair of trousers. The device 1 shown in Fig. 2 may be provided with a clip (not shown) for attaching the device 1 to a belt or clothing. The device 1 in Fig. 2 comprises a "power" or on/off switch 32. Although not shown in fig. 1 it will be appreciated that such power switch 32 may also be present in the device 1 shown in Fig. 1.

In the example of Fig. 3, the device 1 is provided with a wrist-strap 34, so that the device can be arranged to be worn as a wristwatch. In this example the device 1 is arranged to also display time as a regular wristwatch.

Although the functionality of the device 1 according to the invention may be provided by running suitable software code portions on a portable general purpose device such as a personal digital assistant (PDA), portable digital games console or cellular telephone, it is preferred that the device 1 is designed as a dedicated device. The use of the dedicated device provides the advantage that the number of input and output controls is adapted to the specific functioning of the device 1 for psychological monitoring. Hence, simple input controls may be provided adapted for use by e.g. children, elderly, patients and disabled persons. This too improves the compliance with the assessment protocol. Further, providing the dedicated device greatly simplifies the burden to present the questionnaires to the user in real-time.

The device 1 allows for the assessment of moment-to-moment human experiences in the flow of daily life (ESM), allowing not only for the assessment of complicated experiences including affective state, stress, salience, motivation, pain and many others, but also of the context in which the experience arose, allowing for the assessment of human responses in the flow of daily life.

Since the device 1 is an electronic device, it allows for automated replacement of many human interface variables that interfere with generalised application of BMF such as the necessity to use pencil and paper, and the necessity to organise and apply order to pencil and paper output in order to have functional feedback from the data. Hence, prior art cross-sectional questionnaires, e.g. pencil-and-paper, do not accurately inform doctors and therapists about what actually happens in the flow of daily life, yet it is these latter experiences that influence mind and body. Especially pencil-and-paper questionnaires are easily put aside or not answered on a moment that is random to the user, but rather answered on a moment that is convenient to the user. It has been found that presenting the question or questions of the questionnaire to the user using the device 1, a better compliance is achieved, in that the user more often and/or more accurately answers the questions, and hence better complies with the assessment protocol.

The device 1 provides a unique way to assess behavioural and emotional processes at the level of moment-to-moment variation in the flow of daily life, offering new insights to not only professionals, such as physicians, but also to the user, e.g. patient, himself or herself, whose experiences at the moment-to-moment level are not readily accessible for conscious reflection and therefore cannot be elicited using prior art cross-sectional instruments used in psychology, let alone be modified, e.g. therapeutically. The device 1 therefore introduces a whole new dimension of experiential assessment and therapy in medicine and psychology from which not only professionals, but also the user can gain valuable new insights and ways to influence behaviour and/or disease.

The device 1 requires no highly specialised and expensive personnel in the context of specialised treatment centres outside primary care. The device 1 can be applied by nurses, general practitioners, social workers and other professionals. Indeed, the device 1 may be made available as over-the-counter device for use by motivated individuals.

The device 1 offers a cost-effective opportunity to accompany somatic treatments with a behavioural intervention using the electronic device 1 with a semi-human and easily manageable interface.

For these reasons, the potential for the use of the device 1 is extremely high, and may remedy some current deficiencies in medicine including:
a. extremely poor compliance with medication, related to reluctance of patients to cope passively with illness, and exposing themselves to products of the pharmaceutical industry that is, rightly or wrongly, widely suspected of pushing products for non-productive economic gain rather than promoting health;
b. poor results in routine practice of treatments with good results in controlled clinical trials, failure of treatments in routine practice in somatic medicine likely partly resulting from different circumstances that have a large psychological impact, in particular much more and better monitoring of health status during trials, and more attention to patients behavioural and emotional experiences in general (in order to prevent drop-out and withdrawal of consent); and
c. poor results due to unilateral somatic approach resulting in failure to achieve synergistic treatment effects of combined somatic and psychological approaches.

In the area of well-being, a similarly need for the device 1 can be perceived. Achieving insight into one's emotional life, and striving towards an existence directed to what happens in the moment is rapidly conquering the western world. The device 1 is ideally suited to help individuals achieving these goals.

In a more elaborate embodiment, the device 1 is arranged to monitor physical, physiological and/or biochemical parameters of the user. Thereto, the device 1 is provided with at least one sensor arranged to measure a value of such parameters, such as blood pressure, heart rate, body temperature, etc.

Alternatively, or additionally, the device 1 may be provided with connections (e.g. sockets and/or wireless connections) to communicatively connect the device to external sensors.

In one embodiment, the parameters are monitored during answering a questionnaire. It will be appreciated that the parameters may also be monitored during a period of time prior to, preferably contiguous to, the questionnaire being presented to the user. The parameters may also be monitored during a period of time after, preferably contiguous to, the questionnaire being completed by the user. Hence, information obtained from the questionnaire may be combined with information obtained from monitoring the parameters at substantially the same moment in time and/or (just) prior to or after presenting the questionnaire. This combined information increases the options for an operator, e.g. a physician, to assess the mental state of the user.

### Example of a software protocol for the device

The software code portions running on the processor 20 of the device 1 drive a software application on the device 1 that can be used for Experience Sampling (ESM) data collection but also for event based data collection applications. It can also be used to control and optimize interventions using branching. Finally it can be used to design interventions.

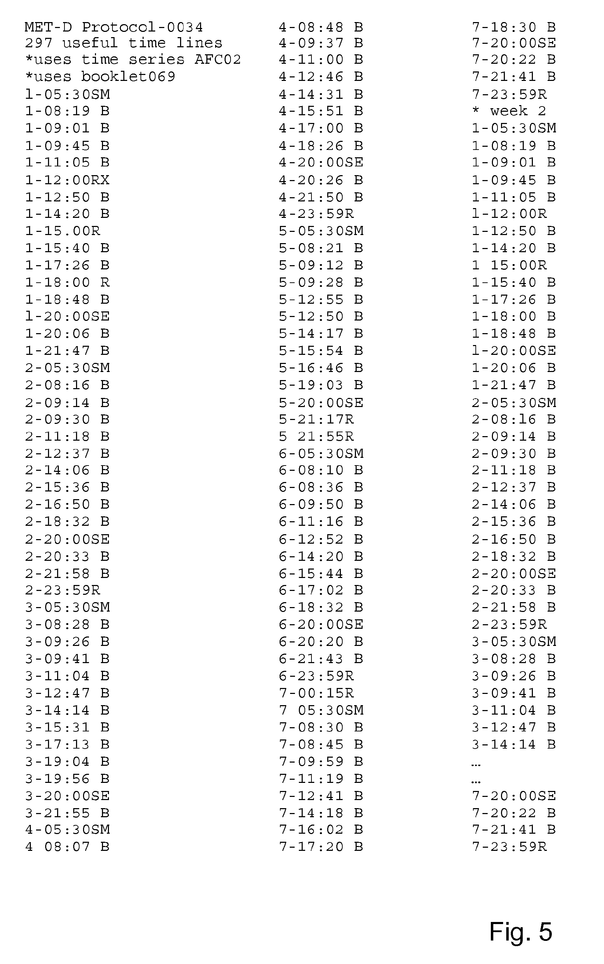

The exemplary software application will search for one or more Protocol files. Such protocol file has the reserved name MET-D Protocol-[subcode]" (e.g. MET-D Protocol-0034, see Fig. 5). Optionally, one master protocol file controls the activation of different subprotocols. In this example, the "MET-D Protocol-" part of the filename is a reserved word (case sensitive). The subject name part can be any kind of combinations of numbers and letters up to 16 characters (case sensitive). The protocol is initiated in step 400 of the process executed by the device 1 as demonstrated in the flow chart shown in Fig. 4.

In addition one or more questionnaires labelled "MET-D Questions-[name]" are present in a memo database in the memory 18 of the device 1. In this example, there are two different types of Questionnaire files. The first type is the time-activated ESM questionnaire (see 402 in Fig. 4), to be presented to the user at a moment random to the user, and the second is the event-activated event questionnaire, to be presented to the user at a moment known to the user (see 404 in Fig. 4). The content of these files need not be different.

Further, in this example there are three reserved names for event-questionnaires: "MET-D Questions-Morning"; "MET-D Questions-Evening"; and "MET-D Questions-X". The application will save inputted data in results-files in the memory 18.

The protocol file can contain a number of memo lines. In this example, the name of the protocol file is the first memo line of the protocol file. Here the memo lines start with "*". These memo lines can e.g. contain any information that labels the user.

The time series defining the assessment protocol is preprogrammed in this example. Therefore, any type of series (random, fixed, mixed etc.) can be used. There is a restriction that the time series will be repeated when the number of occurrences defined in the protocol file is exhausted. Here an occurrence is the activation of an ESM questionnaire or event questionnaire. Consequently, the protocol file may contain occurrences that are spread out over several weeks. For a protocol that lasts for 3 weeks, the 4^{th} week will start again at the beginning of the protocol. In multi-week protocols the occurrences of the second week will start again with day 1 (for Monday etc.).

In this example, the protocol file contains a reference to the number of useful datalines (excluding memo lines). The number is the only relevant aspect of the command line. The text will be skipped as a comment.

There is no principal limit to the number of questionnaires to be presented to the user in one day. However, in practice a limit may be posed to the number of questionnaires presented to the user in a day, e.g. not to overload the user with questionnaires. In this example the limit is set to a maximum of 25 beeps each day, 175 on a week. The beeps of the time sampling schemes may relate to selections or killings of event questionnaires or selections of ESM questionnaires. Finally a "Reset" can be defined and should be added periodically for technical reasons.

The beeps will be emitted, by the output 12, at predefined times on specific days of the week (here 1=Monday; 2=Tuesday; 3=Wednesday; 4=Thursday; 5=Friday; 6=Saturday; 7=Sunday). The day indicator is followed by a "-"and the time in 24-hour notation (e.g. a beep at 09:12 on Sunday is "7-09:12"). The time indicator is followed by two entries. The first entry is a space, an "S" (uppercase, for Select), a "K" (uppercase, for Kill) or an "R" (uppercase, for Reset). The last entry of the line is the first letter of the questionnaire that has to be presented at the moment specified by the time indicator. For instance "7-09:12 B" will activate questionnaire "B" (e.g. "MET-D Questions-Beep") (410 in Fig. 4) using a "beeping" alarm.

The uppercase "S" generates a "select alarm" and selects or activates an event questionnaire. An event questionnaire is presented to the user at a moment known to the user. The Morning questionnaire is for instance activated at 5:30 am using "d-05:30SM". In this example, the morning questionnaire will be selectable under a "sun"-icon on the touch screen (selectable e.g. by double click) from that moment on. The uppercase "K" function "kills" a questionnaire. It de-activates for instance the morning questionnaire at 13:00 on day "d" by using "d-13:00KM". From that moment on the morning questionnaire can no longer be selected, and data will be stored indicating that the morning questionnaire has not been activated if relevant.

Morning and Evening questionnaires are default/reserved words. They cannot both be active at the same time. The selection of the evening questionnaire at e.g. 20:00 using "d-20:00SE" will implicitly generate a "kill" for the morning questionnaire ("d-20:00KM") and put the evening questionnaire under a "moon" icon on the touch screen.

Morning and evening questionnaires are day-level event questionnaires in that they are presented to the user once per day on a daily basis (408 in Fig. 4). When the entries of the first page are left blank, the next-page button will generate an abort. This allows the abort of an erroneously selected questionnaire.

Other event questionnaires may be activated by the user himself/herself. Thereto an event questionnaire must be enabled by the protocol. Such event questionnaires enabled with the "S" function will in this example be put under a "lightning" icon (406 in Fig. 4) on touch screen. In this example, there should always be one "X" questionnaire (412 in Fig. 4) for the default event questionnaire (e.g. an open question for memo's). Other event questionnaires are optional and should be enabled by the protocol. The event questionnaires under the "lightning" icon can be enabled (with a "S" function), disabled (using the "K" function - the default X questionnaire will be reenabled) or switched by selecting another event questionnaire. The user may for instance activate an enabled event questionnaire when he/she feels a need to answer the event questionnaire, for instance due to anxiety, craving or the like.

The software application may be arranged to, e.g. randomly, select an event questionnaire from a plurality of questionnaires. Hence, the user knows the moment at which the questionnaire is presented, but does not know in advance which questionnaire will be presented. This may reduce response attrition.

Further, an "R" or reset-event may be defined as a housekeeping procedure at average once for each 20 events.

A space following the time indication in the protocol file will generate a beep and activate the ESM questionnaire selected by the settings on a moment random to the user. Multiple, different ESM questionnaires can be used. E.g. "d-13:34 B" generates a beep at 13:34 on day d of the week (1=Monday; 2=Tuesday;...; 7=Sunday) and activates questionnaire B(ooklet069); a later "d-14:58 C" will generate a beep at 14:58 on day d and activate questionnaire C.

In this example a beep will ring in short bursts lasting approximately one minute and can be aborted by a push on a button 8.i (any button will do) or a tap on the touch screen 4. The beep may be selectable. The beep may also be arranged to automatically be repeated after a predetermined period of time if no button was pushed when the beep rang. Alternatively, or additionally, the questionnaire may be activated by the user at a moment after the beep has rung when the user did not push any button when the beep first rang, e.g. by selecting the appropriate icon on the touch screen.

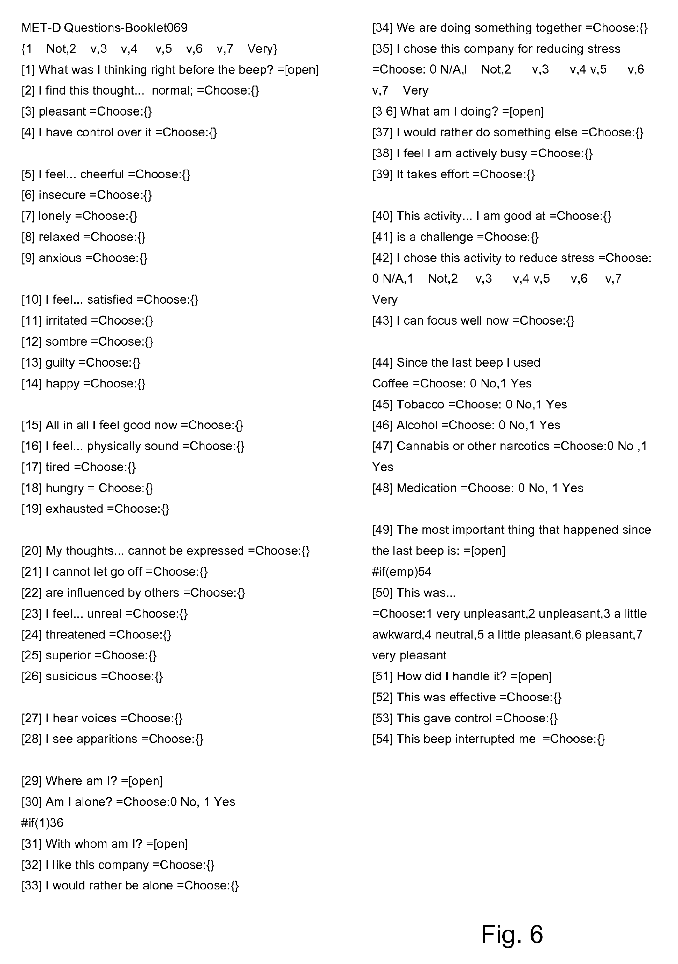

In this example the Questions file is labelled "MET-D Questions-[name]" (e.g. MET-D Questions-Booklet069, see Fig. 6). The "MET-D Questions-" part of the filename is a reserved word (case sensitive). The [name] part can, in this example, be any kind of combinations of numbers and letters up to 20+ characters (case sensitive). In this example, the first character is always a letter. Here, it should be unique because it refers to the entries in the MET-D Protocol file that activates different questionnaires. In this example, the name of the file is the first memo line of the questions file.

In this example, the questions file may contain up to 99 questions and the total length of the file should not exceed 4000 characters, although any number of questions or characters is possible.

The first line after the file name can contain an optional scale-macro. The scale macro contains the default scale format. Here, it is enclosed by parentheses (opening by "{"and closing with "}"). For instance, when most questions are rated on a 7-point Likert scale you can use the string "1 Not,2,3,4 Moderate, 5,6,7 Very" as the default options. The options may be displayed on a pop-up menu as follows:
1 Not
2
3
4 Moderate
5
6
7 Very

It is preferred to label all the options or only the extremes. Labelling extremes and the middle entry often diverts the user from the ordinal structure of the scale and leads to lower frequencies of the unlabelled entries. This problem seems to be less when switching the order of the scale and use a format such as "7 Very,6 | ,5 | ,4 -,3 | ,2 |, 1 Not". The macro will be activated each time the choice options of a question are left blank.

In this example, all questions in the questions file are ordered and the sequence is uninterrupted (except for absolute or conditional jumps). The sequence is labelled between square brackets (e.g. [23] is question "23"). The text of the questions has a free format. All displayable characters are allowed. The line after the question contains the response alternatives. The options in this example are:
=[open]: an open ended question; the system responds by displaying an alphanumeric keyboard on the touch screen;
=[time]: a time entry; the system responds with the time select option. Hour options have one entry (0-23 hours) selectable using the scroll wheel 10 and/or (arrow) buttons 8.i; minute options have a ten-entry (0-5) and a unit-entry (0-9) both selectable by the scroll wheel and/or arrows;
=[cue]/[list]: selector from a list
   - the [cue] can be any word (e.g. "=Choose:");
   - the [list] is a numerical list or contains the default entry "{}", e.g.
      =Choose: 0 No, 1 Yes
      =Choose: 1 Not,2, 3, 4 Moderate, 5,6,7 Very
      =Choose:{}
   - Options prefixed by an "!" will be set to default ("=Choose:!0 No, 1 Yes").
      Each question can end with an optional line that defines conditional jumps. The format of the line is "#if([range])[question]".
   - The [range] contains "abs" for an absolute or unconditional jump.
   - The [range] can contain "emp" (for empty) to jump when no entry was given in response to an open question.
   - In all other situations a range has to be specified (even when only one option can be selected) (e.g. (1-3) or (0-0)).

In this example, a question with an IF-statement is always the last question of a page, because the IF-statement needs an answer to know which questions have to be activated next. The jump is always to a specific question number (e.g. "#if(1-3)36" jumps to question 36 when the response was 1,2 or 3; e.g. "#if(1-1)36" jumps to question 36 when the response was 1). A jump to a non-existing question ends the questionnaire.

For each response, e.g. for each answered questionnaire, of the user the software application will generate a "MET-D Results-[label]" file, see Fig. 6 for an example. The [label]-part in the header of the file may contain:
- an identification of the user, such as the user's name, the date and the beep time.
- This is followed by "=" and the name of the questionnaire that was answered;
- An optional "*" is added when the questionnaire was responded to as an event. The name of the file with its [label] is the first memo line of the results file, e.g. MET-D Results-Peter : 6-10-00 -18:03 =A*.

In this example, the data records have the following format:
- question number between square brackets;
- response time of that specific question in hh:mm:ss notation with "--:--:--" for any missing values;
- the response to the question. Missing values are labelled "-". The alphanumerical responses to open questions (and/or to [time] questions) are displayed between right and left braces "{I'm home}".

### Examples

In these examples the portable psychological monitoring device is referred to as MET-D.

### Example 1. Psychiatric disorder in combination with medication

Patient D. is diagnosed with a depression by his general practitioner. The depression is not very severe, but severe enough to warrant antidepressant medication. The general practitioner prescribes an antidepressant, but knows that the likelihood of the patient taking the medication as indicated is only around 35%. Therefore, the general practitioner prescribes use of MET-D, which will signal the patient to rate negative and positive mood states, and the contexts which they occur in, at six random time points per day for the next 28 days. The general practitioner tells patient D. that the device will help patient D. getting better by constantly monitoring his mood and providing him feedback about his levels of positive and negative moods, and the contexts in which they occur, in daily life. He explains how the systematic feedback from MET-D will help him get better, and that he should take the antidepressant medication to facilitate this process of gaining control over his mood. Over the next 28 days, patient D. provides input to MET-D at the requested random time points. In addition, in the evening MET-D requests the patient to state whether or not the medication was taken. By constantly focusing on his positive and negative moods, and the context in which they occur, he learns about his mood variations and how to enhance positive and reduce negative affect. In this context, he will be motivated to take his medication as an adjunct to his efforts to change his depression with MET-D. Patient D. checks his progress daily on his computer screen, using MET-Ds ability to transmit data via Bluetooth to his hard drive, and MET-D software that transforms the data into visual displays. After 20 days, patient D's mood is improving, and he attributes this improvement to himself, using MET-D, and the antidepressant as a supportive agent to this effort. After 28 days, patient D. visits his general practitioner. MET-D transmits the data to the computer of the general practitioner, who notes that the patient's mood has lifted and that he has forgotten only three times to take his medication.

### Example 2. Somatic disorder

Patient E. visits her general practitioner. She has, at the age of 39, hypertension that is difficult to control with medication. The general practitioner has noticed that she is a rather "nervous" person. She feels constantly nervous, tense, and worried that things will go wrong. Her mood fluctuates a lot, her concentration is poor and little things make her feel stressed. She tried relaxation once but failed. She is a somewhat chaotic person, with the tendency to negativistic generalising without much attention for the details. The general practitioner suggests that she should get more in contact with the pattern of her responses to daily stressful events, and indicates that he suspects that she generates extreme amounts of negative affect in response to small daily life stressors, and that she should learn to get into contact with this pattern of response and modify it. The general practitioner says that he is not keen to prescribe medication, as it may make her dependent, will interact with her medication for hypertension, and deny her active control over her stress-sensitivity and the way it affects her central nervous system, causing hypertension. Over the next 14 days, patient E uses MET-D, providing information on small daily life stressors and negative and positive affective responses at ten random times per day. When she revisits the general practitioner and MET-D transmits the data, the general practitioner points out on the chart that she is very low in positive affect (she is in fact in the lowest 5% of the population) and generates extremes amount of negative affect in response to small daily life stressors. He also notes that the rare instances of positive affect are those when she has "quality time" with her two daughters and that the negative affective responses typically occur at work. The general practitioner loads a protocol file in MET-D that will systematically stimulate the patient at random times during the day to be consciously mindful of a positive emotion (i.e. having the experience of a positive emotion rather than having a thought about something pleasant), and schedules her for follow-up after a month to measure her blood pressure. When the patient reports back after a month, her diastolic blood pressure has dropped five points. The chart generated by MET-D indicates that the ratio of negative affect over positive affect has dropped from 90% at the previous visit to 75% now. The patient indicates that MET-D helped her, when she was stressed at work, to be actively mindful of a positive experience with her daughters at home, and thus restore her emotional balance. In fact, MET-D helps her keeping things in perspective and not to be swamped by negative generalisations.

### Example 3. Medication compliance

Patient V., aged 72 years, has peptic ulcer and needs to take a proton-pump inhibitor half an hour before each meal. As he tends to forget his medication before the meal, he takes it during the meal or afterwards instead, with the result that the peptic ulcer does not heal sufficiently and the risk of complications increases. The general practitioner therefore programmes MET-D to remind the patient half an hour before the evening meal to take his medication, and monitors medication use. After using MET-D, correct use of medication increases and the ulcer is finally healed.

### Example 4. Well being

Mrs. D.W., aged 45 years, acquires MET-D as a means to analyse and keep track of her mental life, and to strive for improvement and a more positive outlook on life. Her first goal is to assess and modify her positive to negative affect ratio in daily life, in particular in social situations. Using the feedback of MET-D, she is made aware of activities in daily life that increase her positive affect, and notices that these activities in fact do not correspond with the global impressions she herself had about activities that induce positive affect in daily life. Having learned about this contrast, she now can actively change her life style towards activities that increase happiness. She uses MET-D as constant reminder to strive for improvement and higher order goals. As a second goal, Mrs. D.W. wishes to use MET-D as a means to improve her efficiency at work and in daily life. She is mother of two children and combining work, children, household and hobbies is not always easy and sometimes produces stress. Mrs. D.W. wishes to i) increase her attention for the task that she is doing at the present moment, ii) reduce behaviour resulting in postponement of tasks that need to be done (work-related or household-related tasks), and iii) reduce being distracted in behaviour and thoughts, (such as being distracted by incoming emails, conversation of colleagues, or after work by dwelling on thoughts of today's meetings at work, unfinished work, etc. which will drain energy from her). The MET-D is programmed to focus Mrs. D.W. on what she is doing at the moment, whether she gives that particular task her full attention and to make her aware about occurrences of postponing and being distracted. After several weeks of training, Mrs. D.W. notices improvement in the quality and efficiency of her actions and decreased experience of work stress, while gaining quality time for the family.

### Example 5. Addiction

Mr. S. is a heavy smoker and wishes to quit. The general practitioner programmes MET-D to monitor systematically in the flow of daily life moments of cigarette craving, cigarette use and the circumstances accompanying these behaviours. After a period of monitoring of 2 weeks, the computer charts generated by MET-D indicate that typically craving is preceded by moments of negative affect in response to stressful situations. Smoking follows craving about 90% of the time. Smoking does not generate positive affect in the patient, but does produce a decrease in negative affect. The general practitioner loads a programme in MET-D that uses a dense sampling frame of affective state, in order to provide the patient with a means to prevent the negative affect-craving-smoking cycle and develop an alternative behaviour. The patient's target for the next six weeks is to reduce the proportion of craving-smoking transitions from 90% to 75%. The patient monitors his behaviour daily using MET-D generated computer charts.

## Claims

1. Portable psychological monitoring device, comprising an input, an output, a repository and a processor,
wherein the repository comprises a plurality of questionnaires, each comprising one or more questions relating to a mental state of a user,
wherein the processor is arranged for selecting a first questionnaire of the plurality of questionnaires, and presenting at least one of the questions of the first questionnaire to the user via the output at a first moment that is random to the user, and
wherein the processor is arranged for receiving an answer to the question from the user via the input, and storing the answer into the repository.

2. Device according to claim 1, wherein the processor is arranged for selecting a second questionnaire of the plurality of questionnaires and presenting at least one of the questions of the second questionnaire to the user via the output at a second moment that is known to the user.

3. Device according to claim 1 or 2, wherein the first moment in time and/or the second moment in time is stored in the repository.

4. Device according to any one of the preceding claims, wherein the repository comprises information regarding a time schedule according to which one or more questionnaires are to be presented to the user.

5. Device according to any one of claims 1-4, wherein the device is a dedicated device.

6. Device according to claim 5, wherein the device only comprises software code portions for performing the psychological monitoring, and optionally displaying time.

7. System for psychological monitoring, comprising a portable psychological monitoring device according to any one of claims 1-6, an operator station, and optionally a user station, wherein the portable device is arranged for transmitting data relating to the mental state of the user to the operator station, optionally via the user station.

8. System according to claim 7 as far as dependent from claim 4, wherein the operator station is arranged for modifying the time schedule associated with the information comprised in the repository.

9. Method of assessment of a mental state of a user, comprising
- providing a device according to any one of claims 1-6 to the user,
- selecting a questionnaires comprising one or more questions relating to a mental state of a user,
- having the device present at least one of the questions of the questionnaire to the user at the first moment that is random to the user, and
- receiving an answer to the question from the user using the device.

10. Method of assessment of a mental state of a person having mental state disorders and related disorder phenotypes and being subjected to psychotherapy, possibly Cognitive Behaviour Therapy or Mindfulness Based Therapy, using a portable psychological monitoring device according to any one of claims 1-4.

11. Method of treatment or prevention of mood disorders and related disorder phenotypes, in a person in need thereof, comprising subjecting the person to psychotherapy, possibly Cognitive Behaviour Therapy or Mindfulness Based Therapy, using a portable psychological monitoring device according to any one of claims 1-4, directed to improving the person's ability to experience reward.
